(19) Europäisches Patentamt
European Patent Office
Office européen des brevets

(11) Veröffentlichungsnummer: **0 211 303 B1**

# (12) EUROPÄISCHE PATENTSCHRIFT

(45) Veröffentlichungstag der Patentschrift: **31.01.90**

(51) Int. Cl.⁴: **C07D 307/46**, C11B 9/00

(21) Anmeldenummer: **86109965.3**

(22) Anmeldetag: **21.07.86**

## (54) 2-Acyl-5-methylfurane, ein Verfahren zu ihrer Herstellung und ihre Verwendung.

(30) Priorität: **03.08.85 DE 3527975**

(43) Veröffentlichungstag der Anmeldung: **25.02.87 Patentblatt 87/9**

(45) Bekanntmachung des Hinweises auf die Patenterteilung: **31.01.90 Patentblatt 90/5**

(84) Benannte Vertragsstaaten: **CH DE FR GB IT LI NL**

(56) Entgegenhaltungen:
**US-A- 3 931 245**

**CHEMICAL ABSTRACTS, Band 96, Nr. 3, 18. Januar 1982, Seite 324, Zusammenfassung Nr. 18607v, Columbus, Ohio, US; R. ter HEIDE et al.: "Flavor constituents in rum", & QUAL. FOODS BEVERAGES: CHEM. TECHNOL., [PROC. SYMP. INT. FLAVOR CONF.], 2nd 1981, 1, 183-200ACCINES: MOL. CHEM. BASIS VIRUS VIRULENCE IMMUNOGENICITY, [PAP. CONF.] 1983 (Publ. 1984), 28 000**
**S. ARCTANDER: "Perfume and flavor chemicals (Aroma chemicals)", Band 1, 1969, Montclair, N.J. US**
**LIEBIGS ANNALEN DER CHEMIE, Heft 10, 1985, Seiten 1935-1950, VCH Verlagsgesellschaft, Weinheim, DE; S. Scholz et al.: "Friedel-Crafts-Reaktion von 2-Methylfuran mit gesättigten und alpha,beta-ungesättigten Säureanhydriden"**

(73) Patentinhaber: **Haarmann & Reimer GmbH, Postfach 1253, D-3450 Holzminden(DE)**

(72) Erfinder: **Weyerstahl, Peter, Prof.Dr., Wartburgstrasse 40, D-1000 Berlin 62(DE)**
Erfinder: **Vierk, Gerd, Knüllstrasse 28, D-3470 Höxter(DE)**

(74) Vertreter: **Müller, Gerhard, Dr. et al, BAYER AG Konzernverwaltung RP Patentabteilung, D-5090 Leverkusen 1 Bayerwerk(DE)**

EP 0 211 303 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 2-Acyl-5-methylfurane, ein Verfahren zu ihrer Herstellung und ihre Verwendung als Riechstoffe.

Acyl- und 2-Acyl-X-methylfurane sind bereits aus der Literatur bekannt. So ist in Arctander, "Perfume and Flavor Chemicals" Montclair, N.J. (USA), 1969 unter der Nr. 39 2-Acetyl-furan als ein intensiv-balsamisch-süßer Riechstoff mit tabakartiger, fast narkotischer Schärfe und unter der Nr. 1930 3-Methyl-2-(3-methylbutyryl)-furan als Hauptkomponente des Elsholtziaöls beschrieben; das 3-Methyl-2-(3-methylbutyryl)-furan ist aber wegen seines scharfminzigen, krautigen, rautenartigen Geruchs ohne Bedeutung für die Parfümerie. Weitere Acylfurane wie das 2-Propionylfuran, 2-Butyrylfuran, 2-Isobutyrylfuran, 2-Acetyl-5-methylfuran oder 2-(2-Methylbutyryl)-furan sind als Aromakomponenten z.B. von Kaffee, Rum oder Röstkartoffeln bekannt (siehe S. van Straten, "Volatile Compounds in foods", Zeist (N.L.), 1984). Aus der US-PS 3 943 260 sind ferner die Geschmackseigenschaften von 2-Acetylfuran, 2-Propionylfuran, 2-Butyrylfuran, 2-Valerylfuran, 2-Acetyl-5-methylfuran und 2-Propionyl-5-methylfuran bekannt. Ferner sind aus CA 96 (1982), 18607 v 3-(2-Furyl)-propansäureethyl- und -isopentylester als Komponenten des Headspace-Aromas des Jamaica-Rums und aus der US-A 3 931 245 (2-Furyl)-methylester von Thiolcarbonsäuren als Aromastoffe bekannt.

Es wurde nun jedoch gefunden, daß 2-Acyl-5-methylfurane, deren Acylrest in α-Stellung zur Carbonylgruppe mindestens eine Methylgruppe aufweist, einen besonders blumigen rosen-/geraniumartigen Geruchscharakter z.T. mit einer Frucht-, Holz- oder Jasmon-Note und damit äußerst wertvolle, in der Parfümerie gesuchte Geruchseigenschaften aufweisen. Außerdem wirken diese 2-Acyl-5-methylfurane, deren Acylrest in α-Stellung zur Carbonylgruppe mindestens eine Methylgruppe aufweist als Geruchsverstärker in Riechstoffkombination.

Die Erfindung betrifft daher neue 2-Acyl-5-methylfurane der Formel

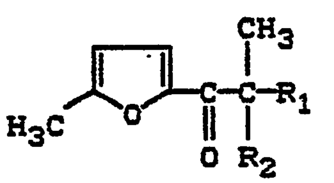

I,

in der

$R_1$ ein Wasserstoffatom oder eine Methylgruppe und

$R_2$ eine Alkylgruppe mit 1 bis 5, vorzugsweise 2 bis 3, Kohlstoffatomen darstellt.

Das Verfahren zur Herstellung der erfindungsgemäßen Verbindungen der Formel I ist dadurch gekennzeichnet, daß man 2-Methylfuran mit Säureanhydriden der allgemeinen Formel

$$(CH_3 - CR_1R_2 - \overset{\overset{O}{\|}}{C} -)_2O \qquad II,$$

in der

$R_1$ und $R_2$ die obengenannte Bedeutung haben, in Gegenwart von Friedel-Crafts Katalysatoren umsetzt.

Die Säureanhydride sind entweder im Handel erhältlich oder können in bekannter Weise [Ber. 57, 423 (1924)] aus den entsprechenden Carbonsäuren mit Acetanhydrid hergestellt werden.

Als Friedel-Crafts Katalysatoren werden vorzugsweise Lewis Säuren wie Zinkchlorid, $BF_3$ (Etherat) und insbesondere Zinntetrachlorid verwendet.

Die Ausgangsprodukte 2-Methylfuran und Säureanhydrid werden in äquimolaren Mengen eingesetzt. Der Lewis Säure-Katalysator wird in Mengen von 0,1 bis 1 Mol-%, vorzugsweise 0,3 bis 0,6 Mol-% bezogen auf Mole 2-Methylfuran angewendet. Die Reaktion wird bei Temperaturen von 60 bis 120°C, vorzugsweise 95 bis 105°C vorgenommen. Die Reaktion kann ohne Lösungsmittel oder in unter den Reaktionsbedingungen inerten Lösungsmitteln wie chlorierten Kohlenwasserstoffen, vorzugsweise 1,2-Dichlorethan durchgeführt werden.

Die erfindungsgemäßen Verbindungen der Formel I stellen wegen ihres blumigen, rosen-/geraniumartigen Geruchscharakters wertvolle neue Riechstoffe dar. Die Erfindung betrifft daher weiterhin die Verwendung der 2-Methyl-5 acylfurane der Formel I als Riechstoffe.

Die erfindungsgemäßen Riechstoffe werden in Kombination mit anderen, an sich bekannten Riechstoffen (Arctander, Perfume and Flavor Chemicals, Montclair, N.J. (USA), 1969) und etherischen Ölen (Arctander, Perfume and Flavor Materials of natural Origin., Elisabeth, N.J. (USA), 1960) angewendet und führen zu Parfümbasen und Riechstoffkompositionen mit ausdruckstarken Noten, die sich hervorragend für die Parfümierung von Fertigprodukten des Aerosol-, Waschmittel- und des Chemisch-Technischen-Sektors, insbesondere aber des Feinparfümerie- und Kosmetiksektors eignen, z.B. für Detergentien, Haarpflegemittel, Schaumbäder, Badesalz, Geschirrspülmittel, Waschpulver, Seifen, Antiperspirans, Puder, Cremes, Rasierwasser, After-Shave-Lotions, Raumluftverbesserer, WC-Reiniger, Raum-Sprays, Antiperspirans-Sprays, Deodorand-Sprays, Körper-Sprays, Sonnenschutzmittel.

In diesen Präparaten werden die erfindungsgemäßen Riechstoffe im allgemeinen in einer Mengen von 0,001 bis 1 Gew.-%, vorzugsweise von 0,001 bis 0,5 Gew.-%, bezogen auf das fertige Präparat, eingesetzt.

Die Herstellung der Parfümkompositionen und parfümierten Produkte erfolgt in üblicher Weise, beispielsweise durch Zusammengeben der Komponenten.

Beispiel 1

8,2 g 2-Methylfuran, 18,6 g 2-Methylbuttersäureanhydrid und 0,13 g Zinntetrachlorid werden bei 100°C 3 Stunden gerührt. Nach dem Abkühlen wird das Reaktionsgemisch mit 50 ml Wasser versetzt und anschließend mit konzentrierter Sodalösung alkalisch gemacht. Die organische Phase wird abgetrennt und die wäßrige Phase mehrmals mit Ether extrahiert. Die vereinigten organischen Phasen werden neutralgewaschen und anschließend das Lösungsmittel im Vakuum abdestilliert. Die Destillation des Rückstandes liefert 11,95 g (= 72 % der Theorie) 2-(2'-Methyl-butyryl) -5-methyl-furan, Kp: 92°C/4 Torr. Der sehr intensive Geruch ist rosig, fruchtig mit einer deutlichen Rosenoxid-Note.

Beispiel 2

8,2 g 2-Methylfuran, 21,4 g 2,2-Dimethylbuttersäureanhydrid und 0,13 g Zinntetrachlorid werden wie in Beispiel 1 beschrieben umgesetzt. Es werden 13,3 g (= 74 % der Theorie) 2-(2',2'-Dimethyl-butyryl)-5-methyl-furan mit einem Siedepunkt von 52°C bei 0,03 Torr erhalten. Der Geruch läßt sich beschreiben als blumige Duftnote mit Rosen-Geranien-Charakter, leicht holziger Unterton, leicht animalisch.

Beispiel 3

8,2 g 2-Methylfuran, 21,4 g 2-Methylpentansäureanhydrid und 0,13 g Zinntetrachlorid werden wie in Beispiel 1 beschrieben umgesetzt. Es werden 14,6 g (= 81 % der Thoerie) 2-(2'-Methyl-pentanoyl)-5-methyl-furan mit einem Siedepunkt von 59°C bei 0,03 Torr erhalten. Der Geruch ist rosig, fruchtig, Geranium, mit einer leichten Jasmon-Note.

Beispiel 4

Eine Riechstoffbase mit einer Grün-Note wird durch Mischen folgender Komponenten hergestellt;

| | |
|---|---|
| 40 Gew.-Teile | 2,4-Dimethyl-1,2,5,6-tetrahydrobenzaldehyd |
| 340 Gew.-Teile | Methyldihydrojasmonat |
| 250 Gew.-Teile | Hydroxycitronellal |
| 360 Gew.-Teile | α-Methyl-3,4-methylendioxyhydrozimtaldehyd |
| 995 Gew.-Teile | |

Der Zusatz von 5 Gew.-Teilen 2-(2'-Methyl-pentanoyl)-5-methyl-furan gestaltet den Grünakkord wesentlich natürlicher und frischer bei gleichzeitiger Anhebung der Geruchsstärke.

Beispiel 5

Ein Rosenakkord wird durch Mischen der folgenden Komponenten hergestellt:

| | |
|---|---|
| 5 Gew.-Teile | Geraniumöl Bourbon |
| 3 Gew.-Teile | Aldehyd C 11 |
| 3 Gew.-Teile | Nectarol |
| 2 Gew.-Teile | Pastinaköl |
| 10 Gew.-Teile | Phenylethylformiat |
| 10 Gew.-Teile | Geranylacetat |
| 80 Gew.-Teile | Citronellol |
| 130 Gew.-Teile | Geraniol |
| 3 Gew.-Teile | Eugenol |
| 2 Gew.-Teile | Jonon |
| 4 Gew.-Teile | Diphenyloxyd |
| 495 Gew.-Teile | Phenylethylalkohol |
| 3 Gew.-Teile | Cyclopentadecanolid (50%ige Lösung in Diethyl-phthalat) |
| 40 Gew.-Teile | Rosacetat |
| 790 Gew.-Teile | |

Der Zusatz von 10 Gew.-Teilen einer 10 %igen Lösung von 2-(2'-Methyl-butyryl)-5-methyl-furan in Diethylphthalat verleiht dem Rosen-Akkord eine natürlichere und frische Kopfnote. Gleichzeitig wird die Duftintensität angehoben.

**Patentansprüche**

1. 2-Acyl-5-methylfurane der Formel

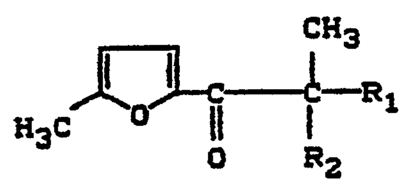

in der
$R_1$ ein Wasserstoffatom oder eine Methylgruppe und
$R_2$ eine Alkylgruppe mit 1 bis 5 Kohlenstoffatomen darstellt.

2. 2-Acyl-5-methylfurane gemäß Anspruch 1, dadurch gekennzeichnet, daß $R_2$ eine Alkylgruppe mit 2 bis 3 Kohlenstoffatomen ist.

3. Verfahren zur Herstellung von 2-Acyl-5-methylfuranen der Formel

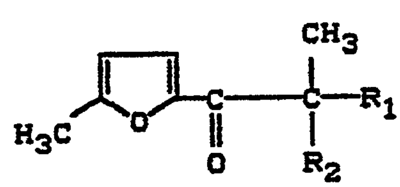

in der
$R_1$ ein Wasserstoffatom oder eine Methylgruppe und
$R_2$ eine Alkylgruppe mit 1 bis 5 Kohlenstoffatomen darstellt,

dadurch gekennzeichnet, daß man 2-Methylfuran mit Säureanhydriden der Formel

$$(CH_3 - CR_1R_2 - \overset{O}{\overset{\|}{C}} -)_2O$$

in der $R_1$ und $R_2$ die vorstehend angegebene Bedeutung haben, in Gegenwart von Friedel-Crafts Katalysatoren umsetzt.

4. Verfahren gemäß Anspruch 1, dadurch gekennzeichnet, daß man als Friedel-Crafts Katalysatoren Lewis-Säuren verwendet.

5. Verwendung der 2-Acyl-5-methylfurane nach Anspruch 1 als Riechstoffe.

## Claims

1. 2-Acyl-5-methylfurans of the formula

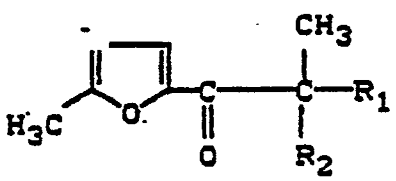

in which

$R_1$ represents a hydrogen atom or a methyl group and

$R_2$ represents an alkyl group having 1 to 5 carbon atoms.

2. 2-Acyl-5-methylfurans according to Claim 1, characterized in that $R_2$ is an alkyl group having 2 to 3 carbon atoms.

3. Process for the preparation of 2-acyl-5-methylfurans of the formula

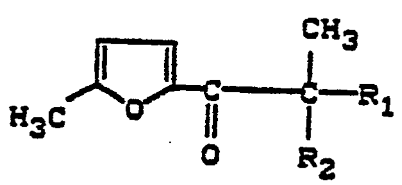

in which

$R_1$ represents a hydrogen atom or a methyl group and

$R_2$ represents an alkyl group having 1 to 5 carbon atoms, characterized in that 2-methylfuran is reacted, in the presence of Friedel-Crafts catalysts, with acid anhydrides of the formula

$$(CH_3 - CR_1R_2 - \overset{O}{\overset{\|}{C}} -)_2O$$

in which

$R_1$ and $R_2$ have the abovementioned meaning.

4. Process according to Claim 1, characterized in that Lewis acids are used as Friedel-Crafts catalysts.

5. Use of the 2-acyl-5-methylfurans according to Claim 1 as aroma chemicals.

## Revendications

1. 2-Acyl-5-méthylfurannes de formule

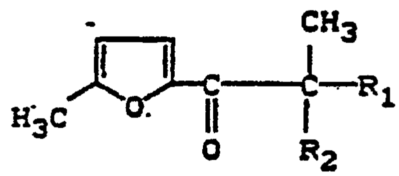

dans laquelle

$R_1$ représente un atome d'hydrogène ou un groupe méthyle, et

$R_2$ représente un groupe alkyle en $C_1$–$C_5$.

2. 2-Acyl-5-méthylfurannes selon la revendication 1, caractérisés en ce que $R_2$ représente un groupe alkyle en $C_2$–$C_3$.

3. Procédé de préparation des 2-acyl-5-méthylfurannes de formule

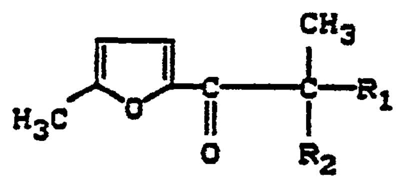

dans laquelle

$R_1$ représente un atome d'hydrogène ou un groupe méthyle, et

$R_2$ représente un groupe alkyle en $C_1$–$C_5$,

caractérisé en ce que l'on fait réagir le 2-méthylfuranne avec des anhydrides d'acides de formule

$$(CH_3 - CR_1R_2 - \overset{O}{\overset{\|}{C}} -)_2O$$

dans laquelle $R_1$ et $R_2$ ont les significations indiquées ci-dessus, en présence de catalyseurs de Friedel-Crafts.

4. Procédé selon la revendication 1, caractérisé en ce que l'on utilise en tant que catalyseurs de Friedel-Crafts des acides de Lewis.

5. Utilisation des 2-acyl-5-méthylfurannes selon la revendication 1 en tant que matières odorantes.